(19) Europäisches Patentamt / European Patent Office / Office européen des brevets

(11) **EP 4 340 729 B1**

(12) **EUROPEAN PATENT SPECIFICATION**

(45) Date of publication and mention
of the grant of the patent:
**29.01.2025 Bulletin 2025/05**

(21) Application number: **21778344.8**

(22) Date of filing: **02.09.2021**

(51) International Patent Classification (IPC):
**A61B 6/03** *(2006.01)*     **A61B 6/04** *(2006.01)*
**A61B 6/00** *(2024.01)*

(52) Cooperative Patent Classification (CPC):
**A61B 6/032; A61B 6/4405; A61B 6/54; A61B 6/547**

(86) International application number:
**PCT/CN2021/116210**

(87) International publication number:
**WO 2023/272930 (05.01.2023 Gazette 2023/01)**

(54) **MEDICAL DEVICE UNDERCARRIAGE AND MEDICAL DEVICE SYSTEM**

FAHRWERK FÜR MEDIZINISCHE VORRICHTUNG UND SYSTEM FÜR MEDIZINISCHE VORRICHTUNG

CHÂSSIS DE DISPOSITIF MÉDICAL ET SYSTÈME DE DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2021   CN 202110729994**

(43) Date of publication of application:
**27.03.2024 Bulletin 2024/13**

(73) Proprietor: **Siemens Shanghai Medical
Equipment Ltd.
Nanhui District
Shanghai 201318 (CN)**

(72) Inventors:
• **WANG, Li**
  **Shanghai 200136 (CN)**
• **LI, Min**
  **Shanghai 201315 (CN)**
• **XIANG, Yin Jie**
  **Shanghai 200080 (CN)**

(74) Representative: **Horn Kleimann Waitzhofer
Schmid-Dreyer
Patent- und Rechtsanwälte PartG mbB
Theresienhöhe 12
80339 München (DE)**

(56) References cited:
US-A1- 2017 215 826     US-A1- 2019 099 140
US-A1- 2020 121 267     US-A1- 2020 245 966

**Description**

**TECHNICAL FIELD**

The present invention relates to a medical device system, and in particular, to a medical device undercarriage.

**BACKGROUND**

During CT scanning, a patient is usually moved by a patient support system (examination table). However, in some special cases, for example, the patient is under surgery or has an open wound, the patient cannot be moved and CT scanning is performed by moving a gantry. This requires a movable gantry undercarriage that can carry and transport the gantry. With this undercarriage, the gantry can be transported outside the scanning room and used in several rooms.

Motion precision is very important for mobile CT and is directly related to image quality of scanning. However, the motion precision of the mobile CT is affected by a variety of factors, including ground flatness and installation precision of a mechanical structure.

US 2020/245966 A1 discloses a medical imaging apparatus and a control method of the same. The medical imaging apparatus including a rotatable gantry in which an X-ray generator configured to generate X-rays and emit the X-rays to an object and an X-ray detector configured to detect the X-rays emitted from the X-ray generator are arranged to face each other, the medical imaging apparatus includes a mover configured to move the medical imaging apparatus, a sensor configured to measure position data according to the movement of the gantry; generate image data based on the detected X-rays, correct the image data based on the measured position data, and generate an X-ray computed tomography (CT) image based on the corrected image data.

US 2017/215826 A1 discloses medical imaging devices, systems, and methods. The medical imaging system may include a movable station and a gantry. The movable station includes a gantry mount rotatably attached to the gantry. The gantry includes an outer C-arm slidably mounted to and operable to slide relative to the gantry mount, an inner C-arm slidably coupled to the outer C-arm and, an imaging signal transmitter and sensor attached to the C-arms. The two C-arms work together to provide a full 360 degree rotation of the imaging signal transmitter. The movable station may include a motion control system and an imaging control system. In embodiments, the motion control system includes omnidirectional wheels for precision controlled-movement of the movable station.

US 2019/099140 A1 discloses an imaging system. The imaging system is operable to acquire and/or generate image data at positions relative to a subject. The imaging system includes a drive system configured to move the imaging system.

US 2020/121267 A1 discloses a mobile imaging system for imaging of patients in medical interventions comprising a ring gantry with a plurality of independently rotating rings whereas a first rotating ring positions an X-ray source with collimator and a second rotating ring positions an image detector such that the region of interest (patient) can be positioned off-centered with respect to the ring center.

**SUMMARY**

In view of this, the present invention provides a medical device undercarriage and a medical device system.

According to a first aspect of the present invention, a medical device undercarriage is provided, including a pair of front wheels, a pair of rear wheels, a first position sensitive detector located between the pair of front wheels, a second position sensitive detector located between the pair of rear wheels, and a controller; and the controller calculates a gear ratio of the rear wheels according to a position of the first position sensitive detector and a position of the second position sensitive detector.

In an embodiment, if a position deviation of the first position sensitive detector is $E_p(n)$, the controller calculates $E_p(n)$ according to the following formula:

$$E_p(n) = K_{pp} * \frac{E_p(n-1)}{dt} + K_{pi} * \frac{\sum_{i=0}^{N} E_p(n-i)}{N * dt} + K_{pd}$$

$$* \frac{\sum_{i=0}^{N} [E_p(n-i) - 2E_p(n-i-1) + E_p(n-i-2)]}{N * dt}$$

where N is a positive integer, and $dt$ is a sampling interval, $K_{pp}$, $K_{pi}$, and $K_{pd}$ are respectively proportional integral differential coefficients of related positions.

In an embodiment, if the position of the first position sensitive detector is $P_1$, the position of the second position sensitive detector is $P_2$, and an angle deviation of the second position sensitive detector is $E_a(n)$, the controller calculates $E_a(n)$ according to the following formula:

$$E_a(n) = \frac{d(P_2 - P_1)}{1000} + E_p(n)$$

where $d(P_2 - P_1)$ is a position difference between the first position sensitive detector and the second position sensitive detector.

In an embodiment, if the gear ratio of the rear wheels is $GR$, the controller calculates $GR$ according to the following formula:

$$GR = 1 + K_{ap} * [E_a(n) - E_a(n-1)] + K_{ai} * E_a(n) * dt + K_{ad}$$
$$* \frac{E_a(n) - 2E_a(n-1) + E_a(n-2)}{dt}$$

where $K_{ap}$, $K_{ai}$, and $K_{ad}$ are respectively proportional integral differential coefficients of related angles.

In an embodiment, $N \in [25, 1000]$.

In an embodiment, the front wheel includes a mobile encoder, a mobile motor, and a mobile drive, and the rear wheel includes a steering encoder, a steering motor, and a steering drive.

According to a second aspect of the present invention, a medical device system is provided, including a medical device and the medical device undercarriage described above, where the medical device is disposed on the medical device undercarriage, and the controller sends the gear ratio of the rear wheels to the medical device.

For the medical device undercarriage and system of the present invention, no track is required to control motion, and no absolutely flat ground is required. The medical device undercarriage and system can be used on a relatively flat ground, have low costs, and are slightly environment-dependent.

**BRIEF DESCRIPTION OF THE DRAWINGS**

To enable a person of ordinary skill in the art to understand the foregoing and other features and advantages of the present invention more clearly, exemplary embodiments according to the present invention are described in detail below with reference to the accompany drawings. In the accompany drawings:

FIG. 1 is a schematic structural diagram of a medical device undercarriage according to an embodiment of the present invention.

FIG. 2 is a functional block diagram of a medical device undercarriage according to an embodiment of the present invention.

FIG. 3 is a schematic diagram of position and angle deviations of a medical device undercarriage according to an embodiment of the present invention.

FIG. 4 is a schematic diagram of motion of a medical device undercarriage according to an embodiment of the present invention.

In the accompany drawings, reference numerals used are as follows:

| | | | |
|---|---|---|---|
| 100 | Medical device undercarriage | 110 | Laser beam |
| 101 | Steering motor | 111 | Mobile drive |
| 102 | Undercarriage plate | 112 | Position deviation |
| 103 | Steering encoder | 113 | Controller |
| 104 | Rear wheel | 114 | Angle deviation |
| 105 | Steering drive | 116 | Path planning |
| 106 | Front wheel | 117 | Medical device |
| 107 | Mobile motor | 118 | Origin |
| 108 | Second position sensitive detector | 120 | First position sensitive detector |
| 109 | Mobile encoder | | |

## DETAILED DESCRIPTION

To make the objective, technical solutions, and advantages of the present invention clearer, the present invention is further described in detail below by using embodiments.

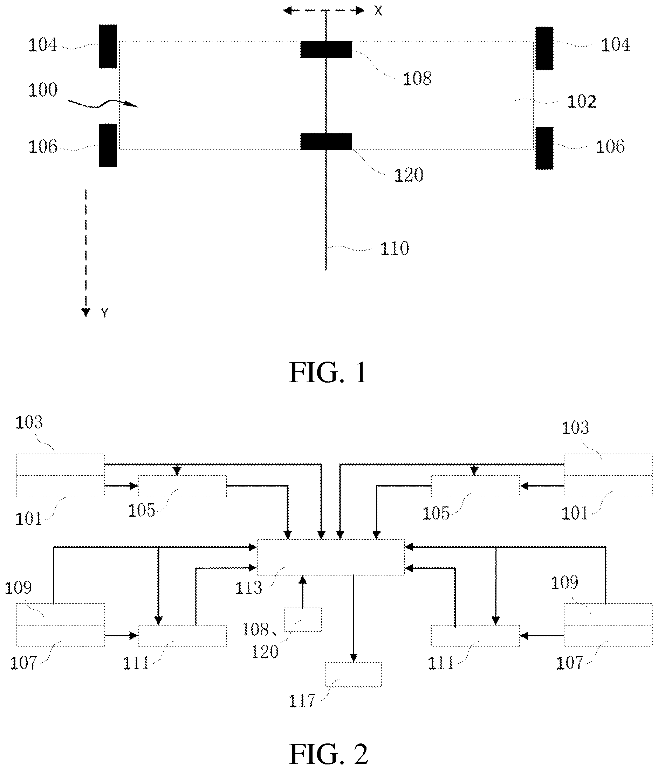

FIG. 1 is a schematic structural diagram of a medical device undercarriage 100 according to an embodiment of the present invention. FIG. 2 is a functional block diagram of the medical device undercarriage 100 according to an embodiment of the present invention. Ideally, the medical device undercarriage 100 will travel in a Y direction, but in fact it will be offset in an X direction. Motion inertia of a gantry is very large, and a back gap exists in a mobile mechanism, so it is difficult to measure a static error. To reduce impact on scanning and reconstruction, a deviation in the X direction should be within 2 mm.

As shown in FIG. 1 and FIG. 2, the medical device undercarriage 100 includes a undercarriage plate 102, a pair of front wheels 106, a pair of rear wheels 104, a first position sensitive detector 120 located between the pair of front wheels 106, a second position sensitive detector 108 located between the pair of rear wheels 104, and a controller 113. The first position sensitive detector 120 and the second position sensitive detector 108 emit a laser beam 110.

As shown in FIG. 2, the front wheel 106 includes a mobile encoder 109, a mobile motor 107, and a mobile drive 111, and the rear wheel 104 includes a steering encoder 103, a steering motor 101, and a steering drive 105. The first position sensitive detector 120 and the second position sensitive detector 108 send their position information to the controller 113. The controller 113 calculates a gear ratio of the rear wheels 104 according to a position of the first position sensitive detector 120 and a position of the second position sensitive detector 108. A medical device 117 may be disposed on the medical device undercarriage 100, and the controller 113 may send the gear ratio to the medical device 117.

Offsets of the two positions on the undercarriage 100 relative to the traveling direction may be obtained from the first position sensitive detector 120 and the second position sensitive detector 108. The position of the first position sensitive detector 120 is $P_1$, the position of the second position sensitive detector 108 is $P_2$, the position $P_1$ of the first position sensitive detector 120 is a position error, and $P_2 - P_1$ is an angle error.

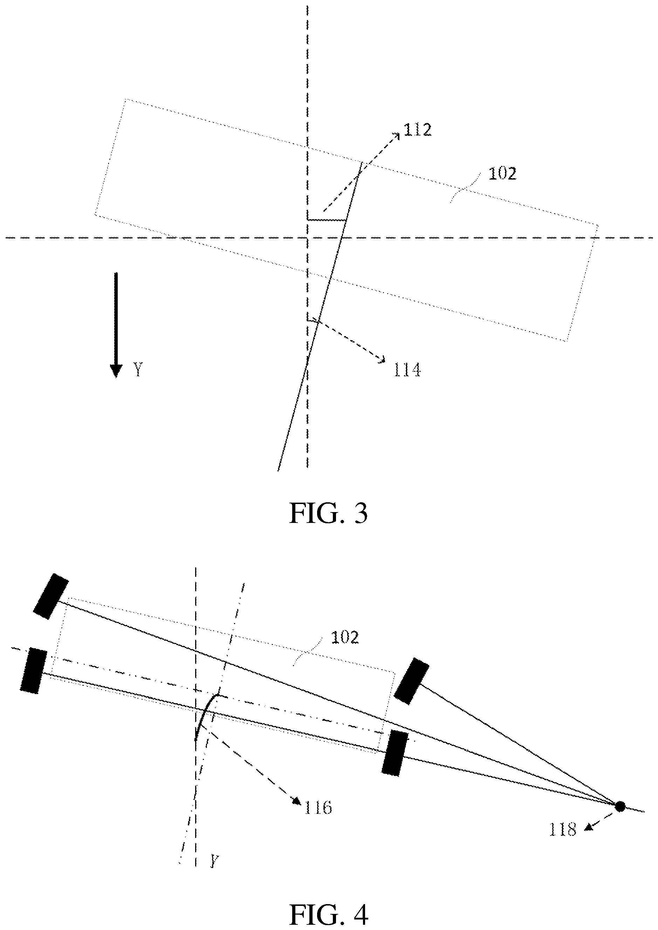

FIG. 3 is a schematic diagram of a position deviation 112 and an angle deviation 114 of a medical device undercarriage 100 according to an embodiment of the present invention.

First, to reduce the position error of $P_1$, the position deviation is used as an input to the first proportional integral differential (PID) loop, and a half-position PID algorithm is used herein. Deviation values of a position sensitive detector at N sampling points are recorded as input deviation values, and N may be a value between 25 and 1000, which can not only increase reliability of deviation correction, but also reduce processor burden.

If a position deviation of the first position sensitive detector 120 is $E_p(n)$, the controller 113 may calculate $E_p(n)$ according to the following formula:

$$E_p(n) = K_{pp} * \frac{E_p(n-1)}{dt} + K_{pi} * \frac{\sum_{i=0}^{N} E_p(n-i)}{N * dt} + K_{pd}$$
$$* \frac{\sum_{i=0}^{N} [E_p(n-i) - 2E_p(n-i-1) + E_p(n-i-2)]}{N * dt}$$

where N is a positive integer, and $dt$ is a sampling interval, $K_{pp}$, $K_{pi}$, and $K_{pd}$ are respectively proportional integral differential coefficients of related positions, and may be obtained through test.

Then, the obtained correction deviation is added to adjustment of the angle deviation, so that adjustment of the angle deviation approaches 0 and reduces the position deviation.

If an angle deviation of the second position sensitive detector 108 is $E_a(n)$, the controller 113 may calculate $E_a(n)$ according to the following formula:

$$E_a(n) = \frac{d(P_2 - P_1)}{1000} + E_p(n)$$

where $d(P_2 - P_1)$ is a position difference between the first position sensitive detector 120 and the second position sensitive detector 108. In this embodiment, $d(P_2 - P_1)$ is in a unit of micron. Therefore, it needs to be divided by 1000 for unit conversion.

A correction amount obtained after the PID loop is compared with an existing speed difference, and further correction is performed. In addition, to increase motion flexibility and smoothness, a rear wheel swivel angle is added, and a deviation angle of the rear wheel is obtained in real time by using a motion center as an origin according to a differential deviation that needs to be corrected.

If the gear ratio of the rear wheels 104 is *GR,* the controller 113 may calculate *GR* according to the following formula:

$$GR = 1 + K_{ap} * [E_a(n) - E_a(n-1)] + K_{ai} * E_a(n) * dt + K_{ad}$$

$$* \frac{E_a(n) - 2E_a(n-1) + E_a(n-2)}{dt}$$

where $K_{ap}$, $K_{ai}$, and $K_{ad}$ are respectively proportional integral differential coefficients of related angles, and may be obtained through test.

FIG. 4 is a schematic diagram of motion of a medical device undercarriage 100 according to an embodiment of the present invention. The rear wheel 104 is deflected around an origin 118, so that a undercarriage plate 102 has path planning 116, thereby returning to the ideal traveling route Y.

A measurement range of a high-precision position sensitive detector (PSD) is +/-17 mm, and resolution is 0.01 mm. In this embodiment, a distance between steering wheels (rear wheels) is 2605 mm, and a diameter of the steering wheel is 198 mm. If only traveling wheels (front wheels) are controlled synchronously in real time, a deviation of 5-7 mm will occur when moving by 1000 mm at 100 mm/s. With the controller of this embodiment, the deviation is reduced to 2 mm.

The present invention further provides a medical device system, including a medical device 117 and the medical device undercarriage 100. The medical device 117 is disposed on the medical device undercarriage 100. The controller 113 sends the gear ratio of the rear wheels 104 to the medical device 117.

For the medical device undercarriage and system of the present invention, no track is required to control motion, and no absolutely flat ground is required. The medical device undercarriage and system can be used on a relatively flat ground, have low costs, and are slightly environment-dependent.

The foregoing descriptions are merely preferred embodiments of the present invention, but are not intended to limit the present invention.

**Claims**

1. A medical device undercarriage (100), comprising a pair of front wheels (106), a pair of rear wheels (104), a first position sensitive detector (120) located between the pair of front wheels (106), a second position sensitive detector (108) located between the pair of rear wheels (104), and a controller (113), wherein
   the controller (113) calculates a gear ratio of the rear wheels (104) according to a position of the first position sensitive detector (120) and a position of the second position sensitive detector (108).

2. The medical device undercarriage (100) according to claim 1, wherein if a position deviation of the first position sensitive detector (120) is $E_p(n)$, the controller (113) calculates $E_p(n)$ according to the following formula:

$$E_p(n) = K_{pp} * \frac{E_p(n-1)}{dt} + K_{pi} * \frac{\sum_{i=0}^{N} E_p(n-i)}{N * dt} + K_{pd}$$

$$* \frac{\sum_{i=0}^{N}[E_p(n-i) - 2E_p(n-i-1) + E_p(n-i-2)]}{N * dt}$$

wherein N is a positive integer, and *dt* is a sampling interval, $K_{pp}$, $K_{pi}$, and $K_{pd}$ are respectively proportional integral differential coefficients of related positions.

3. The medical device undercarriage (100) according to claim 2, wherein if the position of the first position sensitive detector (120) is $P_1$, the position of the second position sensitive detector (108) is $P_2$, and an angle deviation of the second position sensitive detector (108) is $E_a(n)$, the controller (113) calculates $E_a(n)$ according to the following formula:

$$E_a(n) = \frac{d(P_2 - P_1)}{1000} + E_p(n)$$

wherein $d(P_2 - P_1)$ is a position difference between the first position sensitive detector (120) and the second position sensitive detector (108).

4. The medical device undercarriage (100) according to claim 3, wherein if the gear ratio of the rear wheels (104) is *GR*, the controller (113) calculates *GR* according to the following formula:

$$GR = 1 + K_{ap} * [E_a(n) - E_a(n-1)] + K_{ai} * E_a(n) * dt + K_{ad}$$

$$* \frac{E_a(n) - 2E_a(n-1) + E_a(n-2)}{dt}$$

wherein $K_{ap}$, $K_{ai}$, and $K_{ad}$ are respectively proportional integral differential coefficients of related angles.

5. The medical device undercarriage (100) according to claim 2, wherein $N \in [25, 1000]$.

6. The medical device undercarriage (100) according to claim 1, wherein the front wheel (106) comprises a mobile encoder (109), a mobile motor (107), and a mobile drive (111), and the rear wheel (104) comprises a steering encoder (103), a steering motor (101), and a steering drive (105).

7. A medical device system, comprising a medical device (117) and the medical device undercarriage (100) according to any one of claims 1 to 6, wherein the medical device (117) is disposed on the medical device undercarriage (100), and the controller (113) sends the gear ratio of the rear wheels (104) to the medical device (117).

**Patentansprüche**

1. Fahrwerk (100) für eine medizinische Vorrichtung, umfassend ein Paar Vorderräder (106), ein Paar Hinterräder (104), einen ersten positionsempfindlichen Detektor (120), der sich zwischen dem Paar Vorderräder (106) befindet, einen zweiten positionsempfindlichen Detektor (108), der sich zwischen dem Paar Hinterräder (104) befindet, und eine Steuerung (113), wobei die Steuerung (113) ein Übersetzungsverhältnis der Hinterräder (104) gemäß einer Position des ersten positionsempfindlichen Detektors (120) und einer Position des zweiten positionsempfindlichen Detektors (108) berechnet.

2. Fahrwerk (100) für eine medizinische Vorrichtung nach Anspruch 1, wobei, wenn eine Positionsabweichung des ersten positionsempfindlichen Detektors (120) $E_p(n)$ ist, die Steuerung (113) $E_p(n)$ gemäß der folgenden Formel berechnet:

$$E_p(n) = K_{pp} * \frac{E_p(n-1)}{dt} + K_{pi} * \frac{\sum_{i=0}^{N} E_p(n-i)}{N * dt} + K_{pd}$$

$$* \frac{\sum_{i=0}^{N} [E_p(n-i) - 2E_p(n-i-1) + E_p(n-i-2)]}{N * dt}$$

wobei *N* eine positive ganze Zahl ist und *dt* ein Abtastintervall ist, $K_{pp}$, $K_{pi}$ und $K_{pd}$ jeweils Proportional-Integral-Differenzial-Koeffizienten zugehöriger Positionen sind.

3. Fahrwerk (100) für eine medizinische Vorrichtung nach Anspruch 2, wobei, wenn die Position des ersten positionsempfindlichen Detektors (120) $P_1$ ist, die Position des zweiten positionsempfindlichen Detektors (108) $P_2$ ist und eine Winkelabweichung des zweiten positionsempfindlichen Detektors (108) $E_a(n)$ ist, die Steuerung (113) $E_a(n)$ gemäß der folgenden Formel berechnet:

$$E_a(n) = \frac{d(P_2 - P_1)}{1000} + E_p(n)$$

wobei $d(P_2 - P_1)$ eine Positionsdifferenz zwischen dem ersten positionsempfindlichen Detektor (120) und dem zweiten positionsempfindlichen Detektor (108) ist.

4. Fahrwerk (100) für eine medizinische Vorrichtung nach Anspruch 3, wobei, wenn das Übersetzungsverhältnis der Hinterräder (104) *GR* ist, die Steuerung (113) *GR* gemäß der folgenden Formel berechnet:

$$GR = 1 + K_{ap} * [E_a(n) - E_a(n-1)] + K_{ai} * E_a(n) * dt + K_{ad}$$
$$* \frac{E_a(n) - 2E_a(n-1) + E_a(n-2)}{dt}$$

wobei $K_{ap}$, $K_{ai}$ und $K_{ad}$ jeweils Proportional-Integral-Differenzial-Koeffizienten zugehöriger Winkel sind.

5. Fahrwerk (100) für eine medizinische Vorrichtung nach Anspruch 2, wobei $N \in [25, 1000]$.

6. Fahrwerk (100) für eine medizinische Vorrichtung nach Anspruch 1, wobei das Vorderrad (106) einen mobilen Codierer (109), einen mobilen Motor (107) und einen mobilen Antrieb (111) umfasst und das Hinterrad (104) einen Lenkcodierer (103), einen Lenkmotor (101) und einen Lenkantrieb (105) umfasst.

7. Medizinisches Vorrichtungssystem, umfassend eine medizinische Vorrichtung (117) und das Fahrwerk (100) für eine medizinische Vorrichtung nach einem der Ansprüche 1 bis 6, wobei die medizinische Vorrichtung (117) auf dem Fahrwerk für eine medizinische Vorrichtung (100) angeordnet ist und die Steuerung (113) das Übersetzungsverhältnis der Hinterräder (104) an die medizinische Vorrichtung (117) sendet.

**Revendications**

1. Châssis de dispositif médical (100), comprenant une paire de roues avant (106), une paire de roues arrière (104), un premier détecteur sensible à la position (120) situé entre la paire de roues avant (106), un deuxième détecteur sensible à la position (108) situé entre la paire de roues arrière (104), et un contrôleur (113), dans lequel :
le contrôleur (113) calcule un rapport de transmission des roues arrière (104) en fonction d'une position du premier détecteur sensible à la position (120) et d'une position du deuxième détecteur sensible à la position (108).

2. Châssis de dispositif médical (100) selon la revendication 1, dans lequel, si un écart de position du premier détecteur sensible à la position (120) est $E_p(n)$, le contrôleur (113) calcule $E_p(n)$ suivant la formule suivante :

$$E_p(n) = K_{pp} * \frac{E_p(n-1)}{dt} + K_{pi} * \frac{\sum_{i=0}^{N} E_p(n-i)}{N * dt} + K_{pd}$$
$$* \frac{\sum_{i=0}^{N} [E_p(n-i) - 2E_p(n-i-1) + E_p(n-i-2)]}{N * dt}$$

dans lequel *N* est un nombre entier positif, et *dt* est un intervalle d'échantillonnage, $K_{pp}$, $K_{pi}$ et $K_{pd}$ sont respectivement des coefficients différentiels intégraux proportionnels de positions relatives.

3. Châssis de dispositif médical (100) selon la revendication 2, dans lequel, si la position du premier détecteur sensible à la position (120) est $P_1$, la position du deuxième détecteur sensible à la position (108) est $P_2$, et un écart angulaire du deuxième détecteur sensible à la position (108) est $E_a(n)$, le contrôleur (113) calcule $E_a(n)$ suivant la formule suivante :

$$E_a(n) = \frac{d(P_2 - P_1)}{1000} + E_p(n)$$

dans lequel $d(P_2 - P_1)$ est une différence de position entre le premier détecteur sensible à la position (120) et le deuxième détecteur sensible à la position (108).

4. Châssis de dispositif médical (100) selon la revendication 3, dans lequel, si le rapport de transmission des roues arrière (104) est *GR,* le contrôleur (113) calcule *GR* suivant la formule suivante :

$$GR = 1 + K_{ap} * [E_a(n) - E_a(n-1)] + K_{ai} * E_a(n) * dt + K_{ad}$$

$$* \frac{E_a(n) - 2E_a(n-1) + E_a(n-2)}{dt}$$

dans lequel $K_{ap}$, $K_{ai}$ et $K_{ad}$ sont respectivement les coefficients différentiels intégraux proportionnels des angles relatifs.

5. Châssis de dispositif médical (100) selon la revendication 2, dans lequel $N \in [25, 1000]$.

6. Châssis de dispositif médical (100) selon la revendication 1, dans lequel la roue avant (106) comprend un codeur mobile (109), un moteur mobile (107) et un entraînement mobile (111), et la roue arrière (104) comprend un codeur de direction (103), un moteur de direction (101) et un entraînement de direction (105).

7. Système de dispositif médical, comprenant un dispositif médical (117) et le châssis de dispositif médical (100) selon l'une quelconque des revendications 1 à 6, dans lequel le dispositif médical (117) est disposé sur le châssis de dispositif médical (100), et le contrôleur (113) envoie le rapport de transmission des roues arrière (104) au dispositif médical (117).

104

100

106

108

102

120

110

X

Y

FIG. 1

103

101

105

109

107

111

113

108、
120

117

103

105

101

109

111

107

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020245966 A1
- US 2017215826 A1
- US 2019099140 A1
- US 2020121267 A1